(19) Europäisches Patentamt

European Patent Office

Office européen des brevets

(11) **EP 0 767 683 B1**

(12) **EUROPÄISCHE PATENTSCHRIFT**

(45) Veröffentlichungstag und Bekanntmachung des Hinweises auf die Patenterteilung:
**10.05.2000  Patentblatt 2000/19**

(21) Anmeldenummer: 95924307.2

(22) Anmeldetag: **21.06.1995**

(51) Int. Cl.$^7$: **A61M 15/00**

(86) Internationale Anmeldenummer:
**PCT/EP95/02410**

(87) Internationale Veröffentlichungsnummer:
**WO 96/00595 (11.01.1996  Gazette 1996/03)**

(54) **AEROSOL-INHALATIONSGERÄT**

AEROSOL INHALER

INHALATEUR A AEROSOL

(84) Benannte Vertragsstaaten:
**AT BE CH DE DK ES FR GB GR IE IT LI LU MC NL PT SE**

(30) Priorität: **29.06.1994 DE 4422710**

(43) Veröffentlichungstag der Anmeldung:
**16.04.1997  Patentblatt 1997/16**

(73) Patentinhaber:
  • **Boehringer Ingelheim Pharma KG**
    **55216 Ingelheim am Rhein (DE)**
    Benannte Vertragsstaaten:
    **BE CH DE DK ES FR GR IT LI LU MC NL PT SE AT**
  • **BOEHRINGER INGELHEIM INTERNATIONAL GmbH**
    **55218 Ingelheim am Rhein (DE)**
    Benannte Vertragsstaaten:
    **GB IE**

(72) Erfinder:
  • **NÖHL, Klaus**
    **D-55128 Ingelheim am Rhein (DE)**
  • **SPRENGER, Harald**
    **D-55218 Ingelheim am Rhein (DE)**

(74) Vertreter:
    **Fuchs Mehler Weiss & Fritzsche**
    **Patentanwälte**
    **Postfach 46 60**
    **65036 Wiesbaden (DE)**

(56) Entgegenhaltungen:
    **WO-A-92/07599**         **WO-A-92/15353**
    **WO-A-92/17231**         **US-A- 5 284 133**

**Beschreibung**

Die Erfindung geht aus von einem Inhalationsgerät für eine zu inhalierende medizinische Substanz gemäß dem Oberbegriff des Anspruchs 1, wie es aus der US-A-5 284 133 bekannt ist.

Ein typischer Einsatzfall für ein solches Gerät ist ein akuter Asthmaanfall. Auf die Betätigung eines entsprechenden Betätigungsorgans gelangt das Aerosol, welches aus einem Treibgas sowie aus der darin befindlichen und in diesem verteilten medizinischen Substanz besteht, aus dem dafür vorgesehenen Vorratsbehälter durch eine Düse in den Luftkanal eines Mundstückes, von wo es aktiv vom Patienten eingeatmet wird. Die Auslösung der Aerosol-Abgabe bzw. der Vernebelung kann elektronisch oder mechanisch erfolgen.

Was bislang nicht zuverlässig erfaßt werden konnte, ist die Tatsache, ob auf Betätigung des Betätigungsorgans hin tatsächlich eine Vernebelung stattgefunden hat oder nicht. Beispielsweise kann dies verhindert sein durch eine Verstopfung der Düse. Selbstverständlich findet auch keine Vernebelung mehr statt, wenn der Vorratsbehälter des Aerosols geleert ist. Dies kann zu Komplikationen führen, da der Patient vermeintlich die Medizin eingeatmet hat, welche für die Bekämpfung eines akuten Anfalls notwendig ist, dies tatsächlich dann jedoch nicht oder nur zum Teil zutrifft. Eine nur teilweise Zuführung der vorgesehenen Dosis führt beim Patient häufig zu dem Verlangen, durch nochmalige Inhalation die Wirkung der medizinischen Substanz zu steigern. Hierdurch kann ein gefährlicher Zustand durch eine Überdosierung eintreten. Darüber hinaus verliert der den Patient betreuende Arzt die Übersicht über die Anzahl der applizierten Dosen innerhalb eines fest vorgegebenen Zeitintervalls, wodurch eine verantwortungsvolle Behandlung von ärztlicher Seite aus überaus erschwert wird.

Gemäß der oben genannten Druckschrift ist eine Vorrichtung vorgesehen, die bei einer Bewegung des Aerosolkanisters in Richtung Düse ein Signal an eine Auswerteelektronik abgibt. Hierdurch wird die Betätigung des Aerosolkanisters elektronisch erläßt. Eine weitere Kontrolleinrichtung in Form eines Drucksensors oder eines Lippensensors soll die Zuverlässigkeit der Aussage, ob ein Inhalationsvorgang stattgefunden hat, erhöhen. Indessen dienen die erfaßten Werte lediglich als Indikatoren dafür, ob Aerosol das Gerät tatsächlich verlassen hat. Hierin liegt aber eine nicht unerhebliche Fehlerquelle.

Aufgabe der vorliegenden Erfindung ist es daher, ein gattungsgemäßes Aerosol-Inhalationsgerät weiterzubilden, bei dem die tatsächliche Freisetzung des Aerosols zuverlässig erfaßt werden kann.

Gemäß weiterer Aspekte der Erfindung sollen eine Reihe von Auswertungen hinsichtlich einiger Behandlungsparameter wie Anzahl der applizierten Dosen, Verbleib von restlichen Dosen im Vorratsbehälter sowie deren Anzeige, aber auch beispielsweise die Überprüfung des Aerosols dahingehend, ob es mit jenem vom Arzt verschriebenen übereinstimmt, möglich werden.

Diese Aufgabe wird gelöst durch das Merkmal des kennzeichnenden Teils des Anspruchs 1.

Vorteilhafte Weiterbildungen ergeben sich aus den Unteransprüchen.

Erfindungsgemäß ist demnach vorgesehen, daß der vorhandenen Auswerteelektronik des Inhalationsgerätes auch Signale von wenigstens einem, im Strömungsbereich der Düse angeordneten thermischen Sensor zugeführt werden.

Der thermische Sensor erlaubt es, aufgrund der Erkennung der Betätigung und der bei der Vernebelung auftretenden Abkühlung des Treibgases, die Vernebelung des Aerosols positiv zu erfassen, dadurch, daß die Abkühlung des Treibgases zur Abkühlung des thermischen Sensors führt und ein eindeutiges elektrisches Signal erzeugt werden kann. Fehlfunktionen wie verstopfte Düsen oder ein leerer Vorratsbehälter des Aerosols können so sicher erkannt werden.

Der Einsatz eines thermischen Sensors in einem Inhalationsgerät ist zwar aus der WO-A-92/07599 bekannt. Bei dem darin offenbarten Inhalationsgerät dient der thermische Sensor allerdings lediglich zum Triggern der Abgabe der medizinischen Substanz, wenn nämlich ein Einatemstrom vom thermischen Sensor detektiert worden ist. Schon aufgrund der Anordnung relativ zur Austrittsdüse des Aerosols in dem bekannten Gerät vermag der bekannte thermische Sensor nicht die tatsächliche Freisetzung des Aerosols zu erfassen.

In einem speziellen und einfachen Ausführungsbeispiel erzeugt die Auswerteelektronik ein Warnsignal bei fehlender Vernebelung und ein Signal, welches die Anzahl der noch im Vorratsbehälter enthaltenen Inhalationsdosen repräsentiert. Diese Signale werden mittels einer geeigneten Anzeige oder, im Fall der Warnung, mit einer akustischen Meldung dem Patienten mitgeteilt. Durch die Überwachung der Vernebelung kann der Anwender gewarnt werden, wenn keine Vernebelung stattgefunden hat, er also keine medizinische Substanz inhaliert hat, wie dies bei einem leeren Vorratsbehälter oder aber insbesondere auch bei einer verstopften Düse der Fall ist.

Gemäß einer weiteren vorteilhaften Weiterbildung ist vorgesehen, daß der thermische Sensor die bei Beginn der Inhalation an ihm vorbeiströmende Luft detektiert, daraus ein entsprechendes Signal erzeugt und an die

Auswerteelektronik leitet, welche ihrerseits ein Signal erzeugt, welches als Koordinationshilfe für den Patienten für den Inhalationsvorgang zur optischen und/oder akustischen Anzeige gebracht wird.

Der Hintergrund für diese Weiterbildung ist der folgende:

Die korrekte Inhalation des Aerosols läuft idealerweise so ab, daß der Patient nach dem Ausatmen das Gerät an den Mund setzt und nach Beginn der Einatmung die Vernebelung durch Drücken des Betätigungsorgans auslöst. Viele Anwender haben aber Probleme, die Atmung und die Auslösung der Vernebelung zu koordinieren. Ein falscher zeitlicher Ablauf von Einatmung und Auslösung setzt jedoch die Präparatwirkung herab und verschlechtert das klinische Ergebnis. Hier setzt die beschriebene Weiterbildung an, indem dem Patienten durch die optische oder akustische Anzeige gleich nach Beginn des Einatmens mitgeteilt wird, daß die Vernebelung unmittelbar bevorsteht. Auf diese Weise wird das klinische Ergebnis der Behandlung deutlich verbessert.

Gemäß einer weiteren Ausführungsform ist vorgesehen, daß ein weiterer thermischer Sensor außerhalb des Strömungsweges des Aerosols angeordnet ist, welcher ein Referenzsignal erzeugt, das ebenfalls zur Auswerteelektronik geführt wird und mit dem Signal vom ersten thermischen Sensor so verknüpft wird, daß das Ergebnis einen Rückschluß auf die abgegebene Aerosolmenge zuläßt. Die Verknüpfung kann beispielsweise innerhalb eines Differenzverstärkers erfolgen, wo der zeitliche Verlauf der Differenz der Temperatur am ersten thermischen Sensor unmittelbar an der Düse und der Umgebungstemperatur gebildet werden kann. Die nachgeschaltete Auswerteelektronik erhält dann dieses Differenzsignal, wobei dessen Integration in geeigneten Grenzen einen Wert liefert, der mit Hilfe einer in einem Speicher der Auswerteelektronik abgelegten Beziehung hin auf die vernebelte Aerosolmenge führt. Durch die quantitative Erfassung kann dem Patienten nach jeder Inhalation exakte Information darüber gegeben werden, ob eine weitere Inhalation erforderlich ist. Bei Unterschreitung einer bestimmten, in Programm der Auswerteelektronik vorgegebenen Menge kann ein Warnsignal erzeugt werden, welches dem Patienten vermittelt, daß zu wenig Wirkstoff inhaliert worden ist. Diese Überwachungsmöglichkeit führt zu einer höheren Anwendungssicherheit der Inhalatoren und zu einer effizienteren Therapie.

Alternativ kann die abgegebene Aerosolmenge dadurch bestimmt werden, daß die Umgebungstemperatur des ohnehin im Bereich der Düse vorgesehenen thermischen Sensors vor der Vernebelung erfaßt, digitalisiert und in der Auswerteelektronik abgespeichert wird. Wird nun das Aerosol während einer Inhalation vernebelt, wird der zeitliche Verlauf der Temperaturänderungen am Sensor erfaßt als sogenanntes Temperaturprofil. Dieses wird digitalisiert und die zuvor gespeicherte Umgebungstemperatur wird davon substrahiert. Die Integration der Differenz in geeigneten Grenzen liefert einen Wert, der mit Hilfe einer im Speicher der Auswerteelektronik abgelegten Beziehung auf die vernebelte Aerosolmenge führt. Die Beziehung zwischen der Ausgangsspannung des Sensorsignalverstärkers und der Temperatur am Sensor kann im Speicher der Auswerteelektronik abgelegt sein.

Etwaige Nichtlinearitäten des Sensors und des Signalverstärkers und damit auch der Einfluß von Arbeitspunktverschiebungen, verursacht durch Umgebungstemperaturänderungen, können rechnerisch in der Auswerteelektronik eliminiert werden.

Umfaßt die Auswerteelektronik gemäß einer noch weiteren Ausführungsform einen Mikrocontroller, so ist diesem vorzugsweise ein Speicher für die Abspeicherung von Daten sowie eine serielle Schnittstelle zugeordnet für eine Kommunikation mit einem externen Computer.

Diese Ausführungsform bietet dem betreuenden Arzt eine auf dem einschlägigen Gebiet völlig neuartige Kontrollmöglichkeit dahingehend, ob der Patient nach seinen Vorgaben tatsächlich die verschriebene Anzahl von Inhalationsdosen inhaliert hat und innerhalb welchen Zeitraumes dies erfolgt ist. Eine Fülle von in den Speicher zu schreibenden Daten und damit verbundenen Funktionen sind möglich. So kann der Arzt beispielsweise auch ein Zeitintervall vorgeben, nach welchem eine weitere Inhalation durchzuführen ist und durch eine im Modul vorhandene Echtzeituhr ein optisches und/oder akustisches Signal erzeugt wird, um den Patienten an die weitere Inhalation zu erinnern.

Die Funktionen können überwacht und ihre Durchführung oder aber ihr Scheitern ebenfalls in dem Speicher abgelegt werden. Bei einem weiteren Besuch bei dem betreuenden Arzt können diese Daten dann über die serielle Schnittstelle ausgelesen und vom Arzt interpretiert werden. Zum Beschreiben bzw. zum Auslesen des Speichers ist das Elektronikmodul über die serielle Schnittstelle - wie schon erwähnt - mit einem externen Computer zu verbinden.

Die Präsenz der Auswerteelektronik beim erfindungsgemäßen Inhalationsgerät ermöglicht weitere Funktionen: gemäß einer weiteren Ausführungsform kann der Vorratsbehälter für das Aerosol als Austauschteil ausgebildet sein, so daß er lösbar in das Inhalationsgerät einsetzbar ist. Die Außenseite des lösbaren Vorratsbehälters kann optisch kodierte Flächen aufweisen, wie sie beispielsweise bekannt sind bei der sogenannten DX-Kodierung von Kleinbildfilmpatronen in der Fotografie. Die Auswerteelektronik ist dann so ausgebildet, daß sie die in diesem Code enthaltene Information dekodieren kann. Diese Informationen können beispielsweise die Angaben über den Wirkstofftyp der medizinischen Substanz im Aerosol sowie dessen Verfallsdatum umfassen. Diese Daten können ausgelesen werden und in den Mikrocontroller der Auswerteelektronik mit beispielsweise den vom Arzt eingegebenen Angaben hinsichtlich des Wirkstofftyps verglichen werden. Bei Abweichungen kann ein Alarmsignal ausgelöst werden. Die bereits angesprochene Echtzeituhr im Elektronikmodul kann herangezogen werden, um das Verfallsdatum der medizinischen Substanz im Aerosol zu überprüfen und bei Überschreitung desselben Alarm auszulösen.

Bei dieser Ausführungsform kann der austauschbare Vorratsbehälter auch selbst über einen elektronischen Speicher verfügen, wobei die in ihm gespeicherten Daten von der Auswerteelektronik ausgelesen werden können bzw. diese Daten in den Speicher schreiben kann. Dieser Speicher kann zusätzlich zu der angesprochenen optischen Kodierung vorgesehen sein oder aber auch nur für sich genommen. Zum Datenaustausch zwischen Inhalator und einem externen Computer, der beispielsweise beim behandelnden Arzt steht, kann ein steckbares EEPROM-Modul eingesetzt werden, welches ohne Energieversorgung seine Information behält. Der externe Computer verfügt über einen Adapter, über den Daten in das EEPROM-Modul geschrieben und auch aus diesem gelesen werden können. Über dieses Speichermodul können einerseits dem Gerät die Information über den Wirkstofftyp und die Behandlungsvorschrift mitgeteilt werden und andererseits legt das Gerät selbst die Ist-Therapiedaten hier ab.

Bei einer weiteren Ausführungsform kann am Mundstück des Inhalators ein Sensorkontakt vorhanden sein. Das Signal dieses Sensorkontaktes wird der Auswerteelektronik zugeführt. Damit kann geprüft werden, ob der Patient bei korrekter Vernebelung die medizinische Substanz tatsächlich erhalten hat. Es wird verhindert, daß eine an sich korrekte Vernebelung, die in den freien Raum erfolgte, also den Patienten überhaupt nicht erreicht hat, im Speicher als applizierte Dosis eingetragen wird.

Es sei an dieser Stelle nochmals hervorgehoben, daß Voraussetzung für alle diese erwähnten Kontrollmöglichkeiten die Ausstattung des Inhalationsgerätes mit einem thermischen Sensor unmittelbar an der Düse, wo das Aerosol vernebelt wird, ist.

Das eingangs erwähnte Elektronikmodul weist erfindungsgemäß ein eigenes Gehäuse auf, welches an das Inhalationsgerät lösbar befestigbar ist, wobei es von sich abragend den wenigstens einen thermischen Sensor, welcher in unmittelbarer Nähe der Düsenöffnung im Inhalationsgerät anzuordnen ist, aufweist, derart, daß der Sensor nach Herstellung der Verbindung dicht an der Öffnung der Düse anliegt. Das Elektronikmodul kann unabhängig vom Inhalator gefertigt und geprüft werden. Erst beim letzten Fertigungsschritt wird das Elektronikmodul an das Gerät angeklippst und das Komplettgerät endgeprüft.

Gemäß einer vorteilhaften Weiterbildung ist auch der erwähnte zweite thermische Referenzsensor an dem Elektronikmodul angeordnet, wodurch die gesamte Einheit sehr kompakt wird, da dieser zweite Sensor nicht etwa am Inhalationsgerät anzubringen ist, zu dem darüber hinaus noch eine elektrische Verbindung hergestellt werden müßte.

Der thermische Sensor bzw. die thermischen Sensoren sind vorzugsweise NTC-Widerstände, Thermistoren oder Oberflächen-Thermoelemente, also Elemente, die ihre elektrischen Eigenschaften bei einer Temperaturänderung in auswenbarer Weise ändern.

Die Auswerteelektronik kann durch einen Mikrocontroller realisiert werden, oder aber als kundenspezifischer Schaltkreis, in welchem alle erforderlichen elektronischen Funktionen integriert sind, ausgeführt werden. Diese Ausführung ermöglicht es, den Platzbedarf für die Auswerteelektronik und den Strombedarf und wegen der damit verbundenen Reduzierung des Batterievolumens, den gesamten Volumenbedarf für das Elektronikmodul zu reduzieren. Die Platine des Elektronikmoduls kann vorteilhafterweise in SMD-Technik ausgeführt sein. Der Mikrocontroller kann aus Platz- und Kostengründen ohne IC-Gehäuse in Chip On Board (COB)- oder per Tape-Automated Bonding (TAB)-Technik bestückt werden. Die Anzeigeeinheit und die notwendigen Batterien werden über einen geklippsten Kunststoffrahmen auf der Platine fixiert und über Leitgummi bzw. Kontaktfedern elektrisch mit dieser verbunden.

Ein Ein- und Ausschalten des Gerätes kann ohne zusätzlichen Bedienungsschritt durch Abziehen bzw. Stecken der Verschlußkappe des Inhalationsgerätes erfolgen. Bleibt die Kappe länger geöffnet, schaltet sich die Elektronik nach einer bestimmten festgelegten Zeit selbständig in einen sogenannten "Sleep Mode" und wird durch Auslösen einer Inhalation automatisch geweckt. Bei aufgesteckter Verschlußkappe kann eine mechanische Verriegelung vorgesehen sein, welche eine Auslösung der Vernebelung unmöglich macht.

Die Anzeigeeinheit kann vorzugsweise ein LCD mit einer 3-stelligen 7-Segment-Anzeige sowie einem Symbol für die Tagesdosiswarnung, Batteriewarnung und Anzeige eines Gerätefehlers sein.

Wird ein neu befüllter Vorratsbehälter des Aerosols in dem Inhalationsgerät eingesetzt, so muß am Elektronikmodul ein Reset-Taster betätigt werden, wobei dann die Restdosis auf einen Wen gesetzt wird, der einer Behälterfüllung entspricht. Diese Funktion kann gemäß einer weiteren Ausführungsform auch mittels der Vorrichtung zur Erkennung der Betätigung, die sowohl beim Betätigen des Betätigungsorgans, als auch beim Behälterwechsel ein Signal an die Auswerteelektronik gibt, realisiert werden. Der Reset-Taster wird dann nicht benötigt.

Der Inhalationsvorgang beim erfindungsgemäßen Inhalationsgerät kann im übrigen elektronisch ausgelöst werden. Die hierfür erforderliche Elektronik kann ebenfalls in dem Elektronikmodul untergebracht sein. Fehlauslösungen werden weitgehend durch einen Sensorkontakt am Mundstück verhindert.

Die weiter oben erwähnte elektronische Erkennung der Einatemphase durch den thermischen Sensor im Strömungsbereich der Düse kann auch dazu benutzt werden, die Vernebelung atemgetriggert voll automatisch auszulösen. Dazu gibt die Auswerteelektronik dann nach entsprechender Signalauswertung ein Signal zu dem Betätigungsorgan des Inhalationsgerätes und löst so die Vernebelung aus. Die Koordination wird dann vollständig vom Gerät übernommen. Der Patient muß lediglich das Gerät ansetzen und einatmen.

Im übrigen kann die Auswertung des Sensorsignals auch in der Weise erfolgen, daß die analogen Signale nach Verstärkung sofort digitalisiert werden, die Funktion der oben beschriebenen Komparatoren vom Programm der Auswerteelektronik übernommen wird und die entsprechenden Schaltpegel und Verzögerungszeiten vom Programm bestimmt werden.

Nachfolgend wird das erfindungsgemäße Inhalationsgerät anhand eines Ausführungsbeispiels näher erläutert. Hierbei zeigt:

Fig. 1        eine Vorderansicht des Inhalationsgerätes mit seitlich angeklippstem Elektronikmodul,

Fig. 2        eine Seitenansicht des Inhalationsgerätes aus Fig. 1,

Fig. 3        die Aufsicht auf das Gerät gemäß den Figuren 1 und 2,

Fig. 4        ein Prinzipschaltbild des Elektronikmoduls,

Fig. 5a bis 5c    zeitliche Verläufe von Ausgangssignalen, und

Fig. 6        einen zeitlichen Verlauf der Integration des Differenzsignals zwischen dem thermischen Sensor an der Düsenöffnung und dem Referenzsensor, und

Fig. 7        weitere zeitliche Verläufe von Ausgangssignalen, bei denen die Einatemphase elektronisch ausgewertet wird.

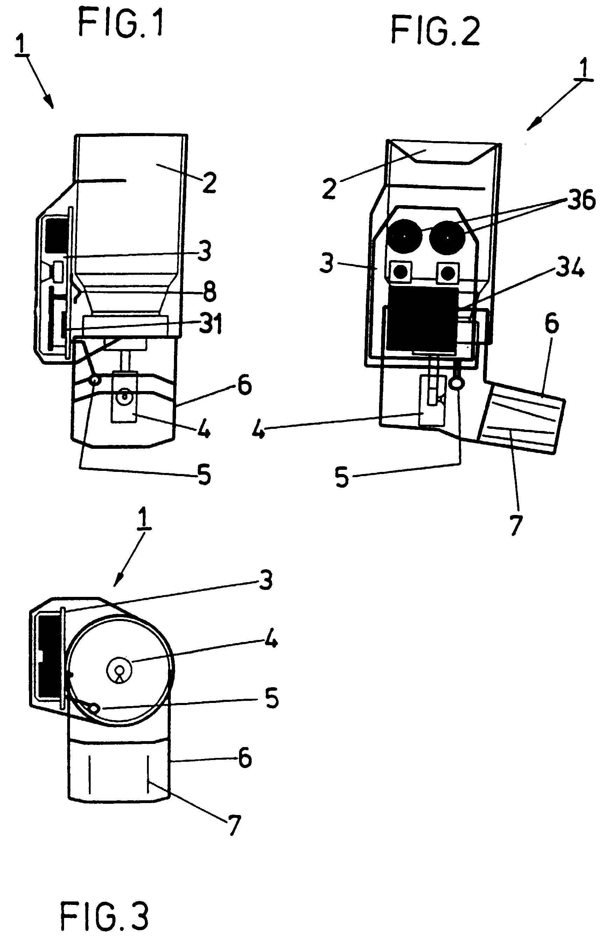

Die Figuren 1 bis 3 zeigen Ansichten des erfindungsgemäßen Inhalationsgerätes, wobei dies lediglich Prinzipdarstellungen sind und tatsächlichen Schnittansichten nicht entsprechen.

Das erfindungsgemäße Inhalationsgerät 1 weist einen Vorratsbehälter 2 für ein Aerosol auf. Der Vorratsbehälter 2 ist hier vorliegend lösbar mit einem Gehäuse verbunden, welches ein Mundstück 6 mit darin verlaufendem Luftkanal 7 aufweist. Durch ein hier nicht dargestelltes Betätigungsorgan wird eine Dosis des Aerosols aus dem Vorratsbehälter 2 in die Düse 4 eingelassen, wo es vernebelt wird. Diesen Nebel atmet der Patient durch den Luftkanal 7 ein.

Bei diesem Vorgang bewegt sich der Vorratsbehälter 2 und löst über die Vorrichtung 8 zur Erkennung der Betätigung ein Signal aus, welches der Auswerteelektronik 31 zugeführt wird. Unmittelbar an der Öffnung der Düse 4 ist der erste thermische Sensor 5 angeordnet. Findet nun eine Vernebelung an der Düse 4 statt, so entspannt sich das Treibgas und die Temperatur sinkt im Bereich der Düsenöffnung ab. Diesen Temperaturabfall detektiert der thermische Sensor 5 und erzeugt ein entsprechendes Signal, welches ebenfalls der Auswerteelektronik 31 zugeführt wird.

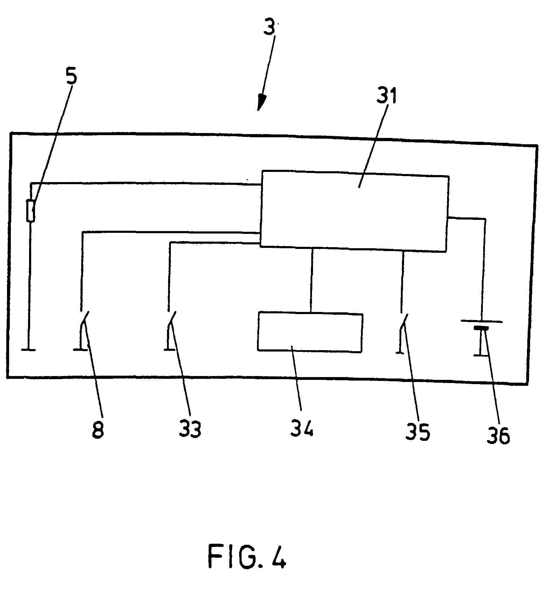

Wie bereits erwähnt, ist das Elektronikmodul 3 vorliegend an das Inhalationsgerät 1 angeklippst und selbständig handhabbar. Es ist von kompakter Bauweise und enthält alle notwendigen Komponenten, von denen in den Figuren 1 bis 3 die Batterien 36 und die optische Anzeige 34 mit Bezugszeichen versehen sind. Die optische Anzeige besteht vorzugsweise aus einer 3-stelligen 7-Segment-LCD-Anzeige. Das Prinzipschaltbild des Elektronikmoduls 3 ist in Figur 4 dargestellt.

Kernstück ist vorliegend eine Auswerteelektronik 31, der das Signal der Vorrichtung 8 zur Erkennung der Betätigung und das Signal vom thermischen Sensor 5 zugeführt wird. Aktiviert wird die Auswerteelektronik erst dann, wenn der Kontakt 33 geschlossen ist, was dann der Fall ist, wenn die Verschlußkappe des Gerätes entfernt worden ist.

Gespeist wird die Schaltung durch die Batterie 36. Die Anzeige erfolgt vorliegend über die bereits erwähnte Anzeigeeinheit 34.

Schließlich ist noch ein Reset-Schalter 35 vorgesehen, der betätigt werden muß, wenn ein neu befüllter Vorratsbehälter in das Inhalationsgerät 1 eingesetzt wird, so daß die verbleibende Restdosis auf einen Wert im Speicher der Auswerteelektronik 31 so gesetzt wird, daß er einer Füllung des Vorratsbehälters entspricht.

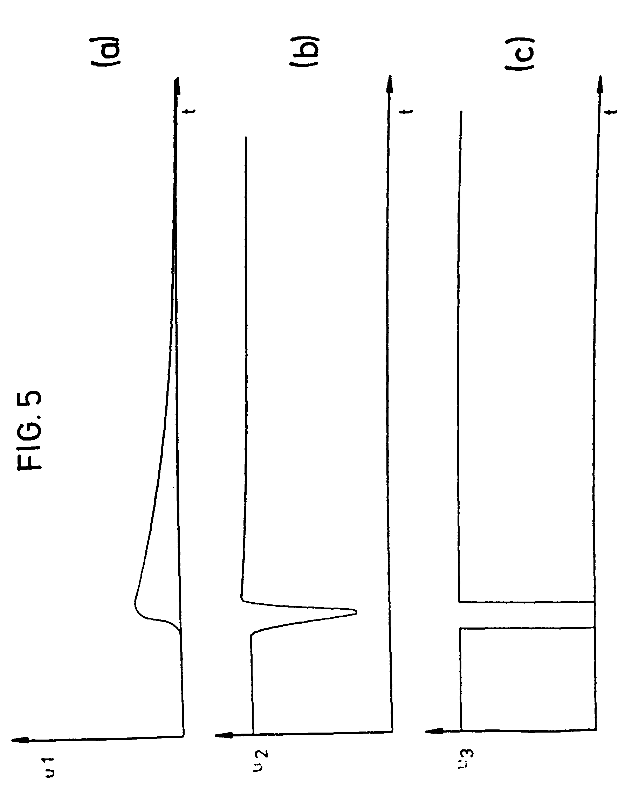

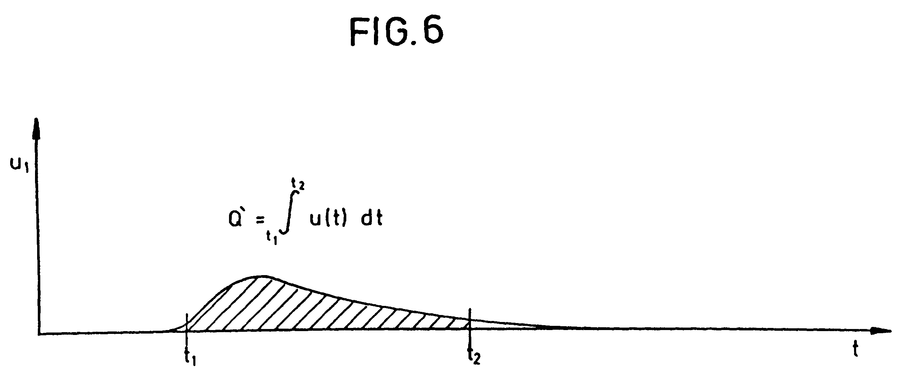

Die Signalaufbereitung wird kurz anhand der Figuren 5 und 6 erläutert.

Fig. 5a zeigt den Verlauf der Spannung $U_1$ am thermischen Sensor 5. Mit der Entspannung des Treibgases an der Düse 4 und mit der damit einhergehenden Abkühlung des Sensors 5 steigt die Spannung $U_1$ zunächst steil an und fällt nach dem Vernebelungsvorgang entsprechend der thermischen Zeitkonstante auf ihren ursprünglichen Wert ab.

Fig. 5b veranschaulicht den zeitlichen Verlauf einer Ausgangsspannung $U_2$ eines frequenzselektiven Verstärkers, an dessen Eingang die Spannung $U_1$ aus Fig. 5a anliegt. Man erkennt, daß $U_2$ im Falle der Vernebelung (Ereignis) einen ausgeprägten Impuls aufweist.

Die Spannung $U_2$ wird beispielsweise einem Komparator zugeführt, der im Ruhezustand eine konstante positive Ausgangsspannung hat und bei korrekter Vernebelung einen negativen Impuls abgibt bzw. im Ereigniszeitpunkt kurzzeitig die Spannung Null annimmt. Die Spannung $U_3$ ist das digitale Eingangssignal für die auswertende Elektronik. Verstärker und Komparator können Teil der Auswerteelektronik sein.

Fig. 6 schließlich zeigt die Möglichkeit der elektronischen Auswertung, wenn ein weiterer thermischer Sensor (nicht dargestellt) als Referenzsensor vorhanden ist, welcher der Umgebungstemperatur ausgesetzt ist. Mit Hilfe eines Differenzverstärkers kann nämlich der zeitliche Verlauf der Differenz zwischen der Umgebungstemperatur und der Temperatur an der Düse 4 im Inhalationsgerät 1 gebildet werden und einem Analog-Digital-Wandler einer im Mikrocontroller enthaltenen Recheneinheit zugeführt werden. Die Integration dieses Signals $U_1$ aus Fig. 6 in geeigneten Grenzen $t_1$ und $t_2$ liefert eine Größe

$$Q' = \int_{t_1}^{t_2} u_1(t)\, dt$$

die mit Hilfe einer in einem ROM-Speicherbaustein der Recheneinheit abgelegten, eindeutigen Beziehung auf die tatsächlich vernebelte Aerosolmenge Q führt.

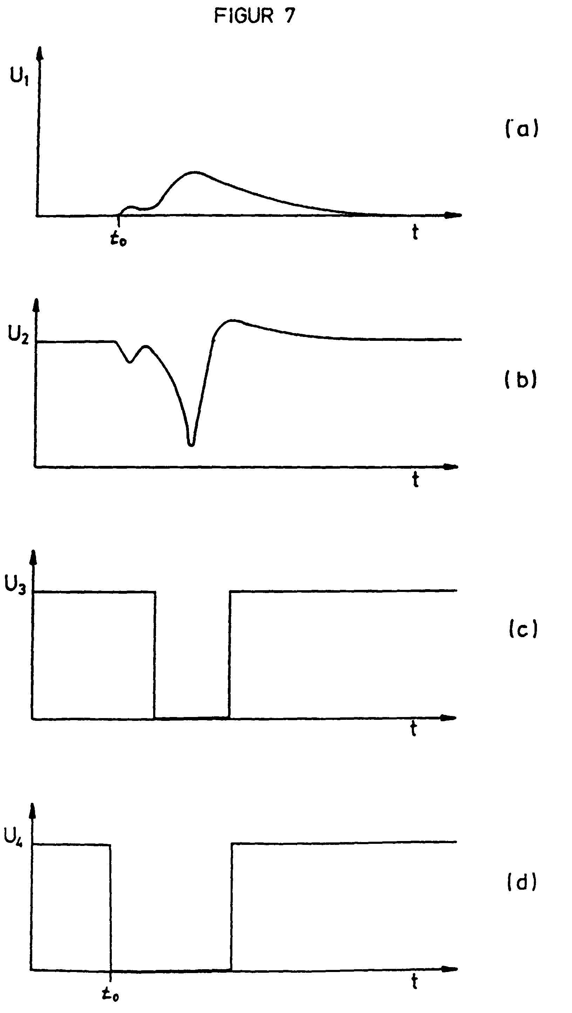

Fig. 7 schließlich zeigt die Signalaufbereitung für den Fall, daß gemäß einer Weiterbildung die Erkennung der Einatemphase elektronisch ausgewertet wird.

Für die Signalaufbereitung wird vorzugsweise der für die Vernebelungserkennung vorhandene frequenzselektive Verstärker benutzt.

In Fig. 7a ist der zeitliche Verlauf der Spannung $U_1$ am thermischen Sensor, in Fig. 7b der Verlauf der Spannung $U_2$ am Ausgang des selektiven Verstärkers, in Fig. 7c der Verlauf der Spannung $U_3$ am Ausgang des Komparators für die Vernebelungserkennung und in Fig. 7d der zeitliche Verlauf der Spannung $U_4$ am Ausgang eines weiteren Komparators für die Dauer einer Einatem- und Inhalationsphase mit korrekter Vernebelung.

Die Spannung $U_1$ am thermischen Sensor weist zunächst einen kleinen Impuls auf, dessen Vorderflanke den Beginn der Einatmung repräsentiert, wenn nämlich die erste Einatemluft über den thermischen Sensor 5 streicht. Die Ausgangsspannung $U_2$ des frequenzselektiven Verstärkers hat hier jedoch schon einen recht ausgeprägten Impuls. Die Spannung wird einem Komparator zugeführt, dessen Ausgangsspannug $U_4$ das digitale Eingangssignal für die Auswerteelektronik 31 ist. Die Vorderflanke der Spannung $U_4$ fällt mit dem Beginn der Einatmung zusammen ($t_0$). Nach einer im Programm der Auswerteelektronik 31 frei definierten Verzögerungszeit zu dieser Flanke gibt die Auswerteelektronik 31 über einen elektromechanischen Wandler (beispielsweise Piezoschwinger) ein akustisches Signal ab.

Der weitere Verlauf der Spannung $U_1$, nämlich in einem stärker positiven Anstieg, und der Spannung $U_2$ mit dem stark negativen Impuls rühren von der Abkühlung des thermischen Sensors 5 durch die auf die Auslösung folgende, korrekte Vernebelung des Aerosols her. Die Ausgangsspannung $U_3$ des entsprechenden Komparators hat dann einen negativen Impuls, welcher der Auswerteelektronik zugeführt wird.

Für den Patienten erleichtert sich die Handhabung dahingehend, daß er nach dem Ansetzen des Inhalators mit der Einatmung beginnt und nur dann, wenn er ein Signal hört, die Vernebelung durch Betätigung des Betätigungsorgans auslöst. Dadurch wird erreicht, daß der Patient nicht etwa sofort nach Ansetzen des Inhalators die Vernebelung auslöst und dann einatmet oder auslöst, wenn die Einatemphase vorbei ist, sondern korrekt mit der Einatmung auslöst. Hierdurch wird die Präparatwirkung günstig beeinflußt.

Die Erfindung ist nicht auf das hier konkret erläuterte Ausführungsbeispiel begrenzt, sondern umfaßt sämtliche weiter oben beschriebene Möglichkeiten hinsichtlich der Anwendungs- und Auswertemöglichkeiten der erfaßten Daten.

**Patentansprüche**

1. Inhalationsgerät mit

 - einem Vorratsbehälter (2) für ein, aus Treibgas und darin verteilter, zu inhalierender medizinischer Substanz bestehenden Aerosol,

 - mit einem Betätigungsorgan und einem, in seinem Inneren einen Luftkanal aufweisenden Mundstück, wobei nach Betätigung des Betätigungsorgans Aerosol aus dem Vorratsbehälter durch eine Düse in den Luftkanal des Mundstücks zum aktiven Einatmen der medizinischen Substanz tritt,

 - einem Elektronikmodul (3) mit einer Auswerteelektronik (31) zur Funktionsüberwachung,

 - mit einer Anzeigevorrichtung (34) zur Ausgabe des von der Auswerteelektronik (31) erzeugten Auswerteergebnisses, und

 - mit einer Vorrichtung (8) zur Erkennung der Betätigung, wobei deren Signale der Auswerteelektronik zugeführt werden,

 dadurch gekennzeichnet,
 daß der Auswerteelektronik (31) darüberhinaus Signale von wenigstens einem, im Strömungsbereich der Düse (4) angeordneten thermischen Sensor (5) zugeführt werden.

2. Inhalationsgerät nach Anspruch 1, dadurch gekennzeichnet, daß die Auswerteelektronik (31) ein Warnsignal erzeugt, wenn keine Vernebelung stattgefunden hat.

3. Inhalationsgerät nach Anspruch 1 oder 2, dadurch gekennzeichnet, daß der thermische Sensor (5) die bei Beginn der Inhalation an ihm vorbeiströmende Luft detektiert und ein entsprechendes Signal an die Auswerteelektronik (3) leitet, die ihrerseits ein Signal erzeugt, welches zur optischen und/oder akustischen Anzeige als Koordinationshilfe für den Patienten gebracht wird.

4.  Inhalationsgerät nach einem der Ansprüche 1 bis 3, dadurch gekennzeichnet, daß ein weiterer thermischer Sensor außerhalb des Strömungsweges des Aerosols angeordnet ist und ein Referenzsignal erzeugt, welches zur Auswerteelektronik (31) geführt wird und mit dem Signal vom ersten thermischen Sensor (5) so verknüpft wird, daß das Ergebnis einen Rückschluß auf die abgegebene Aerosolmenge zuläßt.

5.  Inhalationsgerät nach einem der Ansprüche 1 bis 3 dadurch gekennzeichnet, daß die abgegebene Aerosolmenge dadurch bestimmt wird, daß die Umgebungstemperatur des thermischen Sensors (5) vor der Vernebelung erfaßt, digitalisiert und in der Auswerteelektronik (31) abgespeichert wird, daß während der Vernebelung der zeitliche Verlauf der Temperaturänderungen als Temperaturprofil erfaßt und digitalisiert wird, und daß aus der Integration der Differenz des letzteren Signals und des Wertes für die zuvor abgespeicherte Umgebungstemperatur in geeigneten Grenzen auf die vernebelte Aerosolmenge geschlossen wird.

6.  Inhalationsgerät nach einem der Ansprüche 1 bis 5, dadurch gekennzeichnet, daß die Auswerteelektronik (31) durch einen Mikrocontroller oder durch einen kundenspezifischen Schaltkreis realisiert ist, dem ein Speicher sowie eine serielle Schnittstelle zugeordnet ist, mittels derer von außen Daten in den Speicher geschrieben werden bzw. aus dem Speicher ausgelesen werden können.

7.  Inhalationsgerät nach einem der Ansprüche 1 bis 6, dadurch gekennzeichnet, daß der Vorratsbehälter (2) lösbar in das Inhalationsgerät (1) einsetzbar ist, und an seiner Außenseite optisch kodierte Flächen aufweist, wobei das Elektronikmodul (3) die im Code enthaltene Information decodieren kann.

8.  Inhalationsgerät nach Anspruch 7, dadurch gekennzeichnet, daß der austauschbare Vorratsbehälter (2) einen elektronischen Speicher aufweist, wobei die in ihm gespeicherten Daten von der Auswerteelektronik (31) gelesen werden können bzw. diese Daten in den Speicher schreiben kann.

9.  Inhalationsgerät nach Anspruch 8, dadurch gekennzeichnet, daß der Speicherbaustein im austauschbaren Vorratsbehälter (2) als EEPROM-Modul ausgebildet ist.

10. Inhalationsgerät nach einem der Ansprüche 1 bis 9, dadurch gekennzeichnet, daß das Signal vom thermischen Sensor (5) so ausgewertet wird, daß es das Betätigungsorgan ansteuert und so den Inhalationsvorgang automatisch triggert.

11. Inhalationsgerät nach einem der Ansprüche 1 bis 10, dadurch gekennzeichnet, daß das Elektronikmodul lösbar an ihm befestigbar ist, welches von sich abragend den wenigstens einen thermischen Sensor (5) aufweist, derart, daß der Sensor (5) nach Herstellung der Verbindung dicht an der Öffnung der Düse (4) anliegt.

12. Inhalationsgerät nach Anspruch 11, dadurch gekennzeichnet, daß auch der zweite thermische Sensor an ihm angeordnet ist.

13. Inhalationsgerät nach Anspruch 11 oder 12, dadurch gekennzeichnet, daß der thermische Sensor (5) bzw. die thermischen Sensoren NTC-Widerstände, Thermistoren oder Oberflächen-Thermoelemente sind.

**Claims**

1.  An inhaler with

    -   a supply container (2) for an aerosol consisting of a propellant and a medicinal substance to be inhaled distributed therein,
    -   with an actuating element and with a mouthpiece having an air channel in its interior where, following the actuation of the actuating element, aerosol passes from the supply container through a nozzle into the air channel of the mouthpiece for the active inhalation of the medicinal substance,
    -   with an electronic module (3) comprising an electronic evaluating unit (31) for function monitoring,
    -   comprising a display device (34) for the output of the evaluation result generated by the electronic evaluating unit (31), and
    -   with a device (8) for recognising actuation, the signals of which are supplied to the electronic evaluating unit,
        characterised in that the electronic evaluating unit (31) is also supplied with signals from at least one thermal sensor (5) arranged in the flow region of the nozzle (4).

2.  An inhaler according to Claim 1, characterised in that the electronic evaluating unit (31) generates a warning signal when no atomization has taken place.

3.  An inhaler according to Claim 1 or 2, characterised in that the thermal sensor (5) detects the air flowing past it at the start of the inhalation and sends a corresponding signal to the electronic evaluating unit (3) which itself generates an optical or acoustic signal as a coordination aid for the patient.

4. An inhaler according to one of Claims 1 to 3, characterised in that a further thermal sensor is arranged outside the flow path of the aerosol and generates a reference signal which is fed to the electronic evaluating unit (31) and is linked to the signal from the first thermal sensor (5) in such manner that the result allows the emitted quantity of aerosol to be deduced.

5. An inhaler according to one of Claims 1 to 3, characterised in that the quantity of aerosol released is determined in that prior to the atomization the environmental temperature of the thermal sensor (5) is detected, digitalized and stored in the electronic evaluating unit (31), that during the atomization the time characteristic of the temperature changes is detected and digitalized as a temperature profile, and that the atomized quantity of aerosol is deduced from the integration of the difference between the latter signal and the value of the previously stored environmental temperature within suitable limits.

6. An inhaler according to one of Claims 1 to 5, characterised in that the electronic evaluating unit (31) is formed by a microcontroller or by a customised circuit which is assigned a memory and a serial interface by means of which data can be input into the memory from the exterior and data can be read out from the memory.

7. An inhaler according to one of Claims 1 to 6, characterised in that the supply container (2) is detachably insertable into the inhaler (1) and on its exterior has optically coded surfaces, while the electronic module (3) can decode the information contained in the code.

8. An inhaler according to Claim 7, characterised in that the replaceable supply container (2) has an electronic memory, while the data stored in said memory can be read out by the electronic evaluating unit (31) and the electronic evaluating unit (31) can input data into the memory.

9. An inhaler according to Claim 8, characterised in that the storage module in the replaceable supply container (2) is formed by an EEPROM module.

10. An inhaler according to one of Claims 1 to 9, characterised in that the signal from the thermal sensor (5) is evaluated in such manner that it activates the actuating element and thus automatically triggers the inhalation process.

11. An inhaler according to one of Claims 1 to 10, characterised in that the electronic module can be detachably secured thereto and comprises, projecting away from itself, the at least one thermal sensor (5) in such a way that after the connection has been formed the sensor (5) fits tightly against the opening of the nozzle (4).

12. An inhaler according to Claim 11, characterised in that the second thermal sensor is also arranged thereon.

13. An inhaler according to Claim 11 or 12, characterised in that the thermal sensor (5) or thermal sensors are NTC resistors, thermistors or surface thermoelements.

**Revendications**

1. Appareil d'inhalation comprenant :

   - un réservoir d'alimentation (2) pour un aérosol constitué d'un gaz propulseur et d'une substance médicale à inhaler qui y est distribuée,
   - un organe de commande et un embout présentant un canal à air dans sa partie intérieure, dans lequel, après actionnement de l'organe de commande, l'aérosol provenant du réservoir d'alimentation entre par une buse dans le canal à air de l'embout pour une inspiration active de la substance médicale,
   - un module électronique (3) comprenant un circuit électronique d'exploitation (31) pour contrôler le fonctionnement,
   - un dispositif d'affichage (34) pour afficher le résultat d'exploitation produit par le circuit électronique d'exploitation (31), et
   - un dispositif (8) pour identifier l'actionnement, dont les signaux sont acheminés au circuit électronique d'exploitation,

   caractérisé en ce que des signaux provenant d'au moins un capteur thermique (5) agencé dans la zone d'écoulement de la buse (4) sont acheminés en outre au circuit électronique d'exploitation (31).

2. Appareil d'inhalation selon la revendication 1, caractérisé en ce que le circuit électronique d'exploitation (31) produit un signal d'alarme lorsqu'il ne se produit pas de nébulisation.

3. Appareil d'inhalation selon la revendication 1 ou 2, caractérisé en ce que le capteur thermique (5) détecte l'air qui lui est acheminé au début de l'inhalation et envoie un signal correspondant au circuit électronique d'exploitation (3), qui produit pour sa part un signal qui est transformé en affichage optique et/ou acoustique comme aide de coordination pour le patient.

4. Appareil d'inhalation selon l'une quelconque des revendications 1 à 3, caractérisé en ce qu'un autre capteur thermique est agencé à l'extérieur de la voie d'écoulement de l'aérosol et produit un signal de référence, qui est acheminé au circuit électronique d'exploitation (31) et est combiné avec le signal issu du premier capteur thermique (5) de telle sorte que le résultat permette de déduire la quantité d'aérosol délivrée.

5. Appareil d'inhalation selon l'une quelconque des revendications 1 à 3, caractérisé en ce que la quantité d'aérosol délivrée est déterminée en saisissant la température ambiante du capteur thermique (5) avant la nébulisation, en la numérisant et en la stockant dans le circuit électronique d'exploitation (31), en saisissant et en numérisant, au cours de la nébulisation, la courbe dans le temps des fluctuations de température ainsi que le profil de température et en établissant, par intégration de la différence entre ce dernier signal et la valeur de la température ambiante stockée précédemment dans des limites appropriées, la quantité d'aérosol nébulisé.

6. Appareil d'inhalation selon l'une quelconque des revendications 1 à 5, caractérisé en ce que le circuit électronique d'exploitation (31) est formé d'un microcontrôleur ou d'un circuit spécifique au client, auquel sont affectées une mémoire ainsi qu'une interface sérielle, au moyen de laquelle on peut transcrire de l'extérieur des données dans la mémoire ou selon le cas les lire dans celle-ci.

7. Appareil d'inhalation selon l'une quelconque des revendications 1 à 6, caractérisé en ce que le réservoir d'alimentation (2) peut être inséré de manière amovible dans l'appareil d'inhalation (1) et présente, sur son côté externe, des surfaces optiquement codées, le module électronique (3) pouvant décoder les informations contenues dans le code.

8. Appareil d'inhalation selon la revendication 7, caractérisé en ce que le réservoir d'alimentation remplaçable (2) présente une mémoire électronique, les données qui y sont mémorisées pouvant être lues par le circuit électronique d'exploitation (31) ou selon le cas ces données peuvent être transcrites dans la mémoire.

9. Appareil d'inhalation selon la revendication 8, caractérisé en ce que le composant de mémoire aménagé dans le réservoir d'alimentation remplaçable (2) se présente sous la forme d'un module EEPROM.

10. Appareil d'inhalation selon l'une quelconque des revendications 1 à 9, caractérisé en ce que le signal provenant du capteur thermique (5) est exploité de manière à commander l'organe de commande et à déclencher ainsi automatiquement l'opération d'inhalation.

11. Appareil d'inhalation selon l'une quelconque des revendications 1 à 10, caractérisé en ce que le module électronique peut être fixé de manière amovible à l'appareil, qui présente, en saillie par rapport à celui-ci, le au moins un capteur thermique (5) de telle sorte que le capteur (5) s'applique étroitement sur l'ouverture de la buse (4) après instauration de la liaison.

12. Appareil d'inhalation selon la revendication 11, caractérisé en ce que le second capteur thermique est également agencé sur l'appareil.

13. Appareil d'inhalation selon la revendication 11 ou 12, caractérisé en ce que le capteur thermique (5) ou selon le cas les capteurs thermiques sont des résistances NTC, des thermistors ou des thermo-éléments de surface.

FIG.1

FIG.2

FIG.3

FIG. 4

FIG. 5

(a)

(b)

(c)

# FIG.6

$$\hat{Q} = \int_{t_1}^{t_2} u(t)\ dt$$

## FIGUR 7

(a)

(b)

(c)

(d)